Europäisches Patentamt

European Patent Office

Office européen des brevets

(19)

(11) Publication number: **0 372 127**
A1

## (12) EUROPEAN PATENT APPLICATION

(21) Application number: 88311358.1

(51) Int. Cl.5: **A61F 9/00**

(22) Date of filing: 30.11.88

Claims 20 to 38 are deemed to be abandoned due to non-payment of the claims fees (Rule 31 (2) EPC).

(43) Date of publication of application:
13.06.90 Bulletin 90/24

(84) Designated Contracting States:
AT BE CH DE ES FR GB IT LI LU NL SE

(71) Applicant: **L'Esperance, Francis A.**
**255 Oakwood Road**
**Englewood New Jersey 07631(US)**

(72) Inventor: **L'Esperance, Francis A.**
**255 Oakwood Road**
**Englewood New Jersey 07631(US)**

(74) Representative: **Milhench, Howard Leslie et al**
**R.G.C. Jenkins & Co. 26 Caxton Street**
**London SW1H 0RJ(GB)**

(54) Beam exposure determining method and apparatus.

(57) For modulating the exposure of a surface to be ablated to an ablative radiation beam so that the surface is ablated to a predetermined curvature profile, the invention proposes the imposition into the beam path of a member opaque to and ablatable by the ablative radiation and having a thickness profile corresponding to the required curvature profile of the surface which is to be ablated. The thickness profile of the inserted member determines the amount of beam flux that will be incident upon specific areas of the surface to be ablated within a predetermined beam exposure time span, thinner areas of the inserted member producing more rapid breakthrough of the ablative radiation than thicker areas and correspondingly producing greater exposure times at the surface to be ablated. The invention is particularly useful in ophthalmological surgery of the anterior corneal surface, though it has wider application, and encompasses the provision of a range of differently shaped beam inserted selectable by an ophthalmic surgeon to achieve a required curvature correction operation to suit a specific eye malfunction condition.

## BEAM EXPOSURE DETERMINING METHOD AND APPARATUS

### FIELD OF THE INVENTION

This invention generally relates to a method and apparatus for modulating the flux distribution onto a surface to be profiled of an ablative radiation beam and has particular though not exclusive application to ophthalmological techniques for laser surgery of the cornea which involve the profiling of the anterior corneal surface to achieve a desired curvature for correction for example of a detected abnormal eye condition, such techniques being useful not only in the in situ treatment of the corneas of living patients but also in relation to donor surgery where they may for example be utilized for the profiling of a donor cornea prior to transplantation.

### BACKGROUND OF THE INVENTION

The invention will hereinafter be described in relation, to that aspect of ophthalmic surgery which is concerned with operations upon the external surface of the cornea, though as mentioned above the invention is perceived as being of wider application.

Operations of the character indicated include corneal transplants and keratotomies; such operations have traditionally required skilled manipulation of a cutting instrument. But, however keen the cutting edge, the mere entry of the edge into the surface of the cornea necessarily means a wedge-like lateral pressure against body cells displaced by the entry, on both sides of the entry. Such lateral pressure is damaging to several layers of cells on both sides of the entry, to the extent impairing the ability of the wound to heal, and resulting in the formation of scar tissue.

My European patent application No. 84307972 (EP 0151869), includes a background discussion of the effects of various available wavelengths of laser radiation in ophthalmic surgery and, in particular, surgery performed on the anterior surface of the cornea. It is explained that radiation at ultraviolet wavelengths is desirable by reason of its high photon energy. This energy is greatly effective on impact with tissue, in that molecules of tissue are decomposed on photon impact, resulting in tissue ablation by photodecomposition. Molecules at the irradiated surface are broken into small volatile fragments without heating the remaining substrate; the mechanism of the ablation is photochemical, i.e., the direct breakdown of intra-molecular bonds. Photothermal and/or photocoagulation effects are neither characteristics nor observable in ablations at ultraviolet wavelengths, and cell damage adjacent the ablation is insignificant.

The abovementioned and other European patent applications that I have filed, deal with various concepts whereby laser radiation at ultraviolet wavelengths of 200-nm or less are controlled in delivery of laser radiation to the visually used area of the anterial surface of the cornea so as to penetrate the stroma and achieve a predeterminable volumetric removal of corneal tissue, thereby so correctively changing the profile of the anterior surface as to reduce a myopia, or a hyperopia, or an astigmatic abnormality which existed prior to such laser surgery.

These prior applications also deal with sculpturing penetration of the stroma and whilst the present application deals with sculpturing per se, it is to be understood that manipulative, preparatory and other operations upon the cornea in preparation for sculpturing operations are presently preferred to be effected in connection with the sculpturing method and means to be described herein.

The sculpturing technique of my European patent application no. 87306826 may be briefly stated as involving corneal exposure to a laser beam of varying spot size to achieve an ablated change in anterior-surface curvature in the visually used central area of the cornea. Various means have been described to achieve this result, and most conveniently the previously described means have involved microprocessor means to assure a given exposure program in the course of which spot-size area varies as predetermined to achieve a given profile change.

### BRIEF STATEMENT OF THE INVENTION

According to one aspect of the invention there is provided a method of delivering ablative radiation in a predetermined areal flux distribution onto a surface to be ablated, said method comprising introducing into the path of a beam of ablative radiation a member having a predetermined areal thickness distribution and arranging for the beam of radiation to impinge upon said surface after ablation of said member so that the flux delivery distribution is modulated by the thickness distribution of said member.

According to another aspect of the invention there is provided a device for modulating the areal flux distribution within a beam of ablative radiation, said device comprising material opaque to said radiation and having a predetermined areal thickness distribution such that when the device is placed in said beam ablation of said material produces a differential beam breakthrough distribution such as to modulate the areal flux distribution of the breakthrough beam.

As will be appreciated from the following description, the present invention provides a beam modulation technique which depends for its operation upon different breakthrough or burnthrough rates being established in a device inserted into the ablative beam so that for a predetermined beam exposure time a surface to be ablated will receive differential flux levels at different locations thereof.

As described hereinafter, in one application the invention provides an improved method and means for variable-spot-size laser sculpture of the anterior surface of the cornea to achieve an optical improvement of the involved eye. A specific advantage of the invention is that this is achieved with simplified techniques which do not require microprocessor control of spot-size variation.

Another specific application of the present invention resides in providing the ophthalmic surgeon with an inventory of differently precharacterized disposable elements in accordance with the invention which can be selected from inventory in accordance with the optical change to be achieved, so that, for example, the surgeon can select, e.g., in diopter-related increments, for plus or minus spherical change and/or for plus or minus cylindrical change, the change he deems necessary to improve the optical performance of a given eye. Thus the invention provides, for example, an inventory of disposable elements adapted for selective placement in the path of laser-beam delivery to the cornea. Each of the disposable elements carries a membrane which is opaque to the laser beam and which is designedly subject to ablation when exposed to the laser beam, the thickness of the membrane being a precharacterized function of local area such that a give laser-beam course of the exposure will require greater or lesser time to locally ablate the membrane and thus permit laser-beam exposure past the membrane and into correspondingly localized ablating impingement with the cornea. Stated in other words, varying-spot size at the cornea is achieved by a time-release function of the membrane, on a predetermined area-controlled basis that is designed to achieve the diopter change specifically identified with each particular selectable element.

Further features of the invention are set forth in the appended claims and will be explained hereinafter with reference to illustrative embodiments shown in the accompanying drawings.

DESCRIPTION OF THE DRAWINGS

Fig. 1 is a simplified view in vertical section showing means for supporting a selected ablatiable membrane element of the invention, for use in laser surgery of a given cornea;

Fig. 1A is a fragmentary sectional view to illustrate a modification;

Fig. 2 is a graphical presentation, to differently enlarged and exaggerated abscissa and ordinate scales, to illustrate profiling of ablatable-membrane thickness to achieve a myopia-reducing result in the context of Fig. 1;

Fig. 3 is a simplified sectional view to illustrate the myopia-reducing sculptured-surface correction achieved with an ablatable-membrane characteristic as in Fig. 2;

Fig. 4 and 5 correspond to Figs. 2 and 3, to illustrate the hyperopia-reducing sculptured-surface correction achieved with an ablatable-membrane characteristic as in Fig. 4; and

Figs. 6 and 7 correspond to Figs. 2 and 4, to illustrate a relationship in the modification of Fig. 1A.

DESCRIPTION OF THE EMBODIMENTS

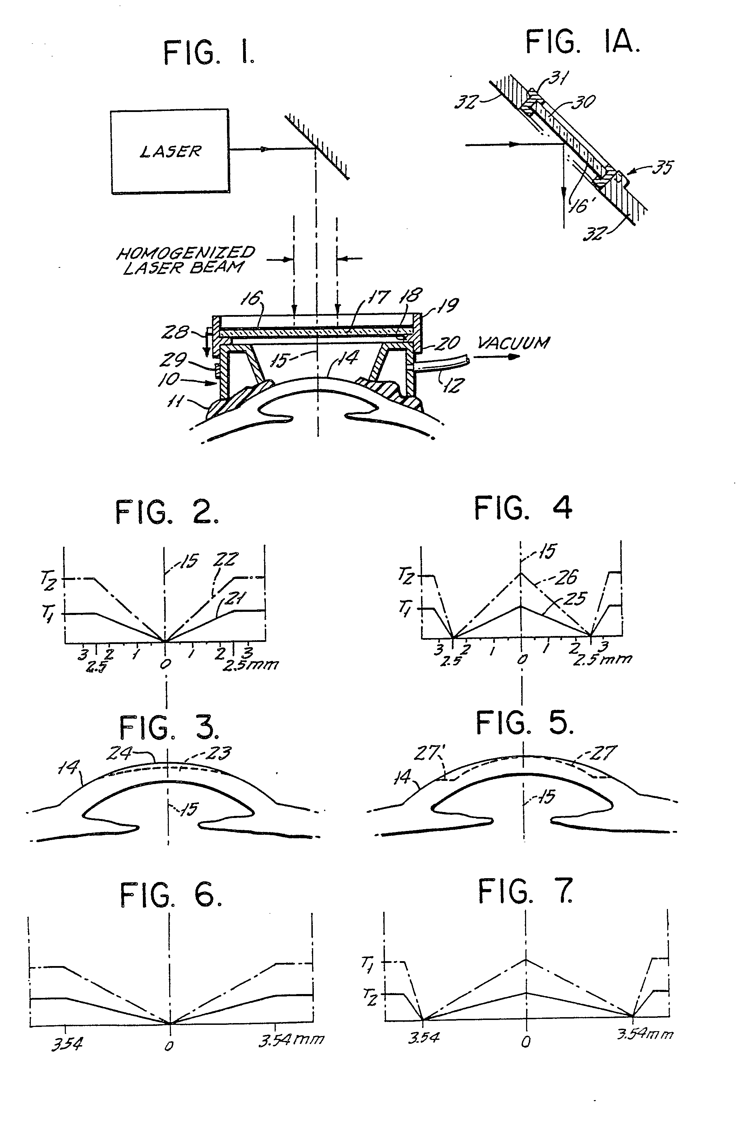

In Fig. 1, the invention is shown in application to an eye-retaining device 10 which may be as described in US Patent No. 4,665,913. Device 10 may thus be a hollow annulus, having a convergent axial- end wall 11 of air-permeable material contoured to engage and retain an eye via a scleral-corneal region. A side-port connection 12 to a vacuum pump enables retention of engagement of wall 11 to the cornea 14 to be operated upon, and, optionally, the device 10 may be fixedly referenced to associated laser apparatus (by means not shown).

Preferably and suitably, the laser apparatus is an excimer laser, committed to pulsed ultraviolet radiation at 200 nanometers or less, as for example provided at 193 nm by an argon-fluoride laser. Preferably also, the output beam of the laser is processed for shaping and for homogeneity of flux distribution, for directional projection of a homogenized coherent beam of circular section (e.g., of 5 or 6-mm diameter) that is centrally aligned with the optical axis 15 of the eye. Means for processing the output of an excimer laser to produce such a homogenized beam of circular section are described in detail in European Patent application no. 88300789 and therefore need not now be further amplified, being identified by suitable legend in Fig. 1.

In accordance with the invention, an ablatable membrane 16 of suitably precharacterized thickness distribution is selectively positionable in the path of laser beam projection on axis 15. Conveniently, the membrane 16 is a coating on the upper surface of a plane-parallel supporting substrate 17 of material that is transparent to the laser beam; a suitable material for this substrate purpose is a synthetic fused silica (e.g., Suprasil, a commercial product of Heraeus-Amersil), or a fluoride compound, such as calcium fluoride, magnesium fluoride, or barium fluoride. In the form shown, substrate 17 is seated upon the inward annular flange 18 of a ring member 19, which is adapted, via a depending flange 20, for concentric engagement to the circular upper rim edge of the eye-retaining device 10.

The membrane 16 is ablatable under action of the laser beam at a rate which preferably (although not necessarily) corresponds with the rate of laser-beam ablative penetration into the stroma of the cornea. Stated in other words, within the central circular area of laser surgery upon the cornea, the maximum thickness of the ablatable membrane 16 is such that, for the maximum extent of stroma penetration involved in a particular corrective procedure, the surgical exposure can be terminated when the entire maximum thickness of exposed membrane is observed to have been ablatively removed. Suitable materials for the ablatable membrane 16 include polyimide, Mylar and poly-(ethylene terephthalate).

Fig. 2 provides illustration of the vertical- section thickness profile for each of two selectively available elements, each of which is configured to effect a myopia reduction. For example, a profile 21 of lesser maximum thickness $T_1$ is seen to reduce from its maximum to zero thickness as a function of decreasing radius about the central axis, for the case of a laser-beam section diameter of 5 mm. And a profile 22 of greater maximum thickness $T_2$ is also seen to reduce from its maximum to zero thickness as a function of decreasing radius about the central axis. The net effective difference between the two profiles is that it will take greater time to reduce the involved central circular area of membrane 16 to a full 5 mm circular bore when membrane 16 is characterized by profile 22, than will be the case when membrane 16 is characterized by the profile 21; this fact translates into accomplishing a greater diopter reduction in cornea curvature for a membrane 16 of thickness profile $T_2$ than is the case for a membrane 16 is thickness profile $T_1$. In both cases, laser-beam exposure should be terminated when the ablated membrane 16 is observed to attain a cylindrical bore which in the present illustrative case will be of 2.5 mm radius, achieving optical-used curvature correction over a 5 mm diameter. In Fig. 3, the newly achieved curvature correction is suggested by dashed line 23, in relation to the more myopic original profile 24 of the cornea 14.

The thickness profiles 25-26 shown for membrane 16 in Fig. 4 are respectively illustrative of what is needed in maximum membrane (16) thickness (a) to achieve hyperopia reduction of lesser degree, using the lesser maximum thickness $T_1$, and (b) to achieve hyperopia reduction of great degree, using the greater maximum thickness $T_2$. In the case of the lesser thickness profile 25, thickness is greatest ($T_1$) at the centre and is a decreasing function of radius to the point of zero thickness at the outer limit of optical correction (e.g., at 2.5 mm radius); in the case of the greater-thickness profile 26, thickness is also greatest ($T_2$) at the centre and is also a decreasing function of radius to the point of zero thickness at the same outer limit of optical correction. If the profiles 25-26 both terminated at the outer limit of optical correction, then the resulting sculpture of cornea 14 would be characterized by a sharply defined outer cylindrical wall, but by utilizing the next 0.5 to 0.75mm increment of radius to taper membrane thickness back to maximum, i.e., radically outward of the outer limit of optical correction, it is possible to materially reduce the sharp-edge nature of such a wall. The ablation upon the cornea thus produces the hyperopia-reduced profile 27 (Fig. 5) out to the illustrative outer limit of optical correction, and a gently beveled annulus 27' of relatively smooth transition to the remaining outer unexposed area of the cornea. It will be understood that to produce the "beveled" hyperopia-reducing result described in connection with Figs. 4 and 5, it is necessary to adjust the circular section of the laser beam to a slightly larger diameter (e.g., 6.5 to 7 mm) than for the case of myopia-reduction (Figs. 2 and 3), but such sectional-area selection is among the capabilities of apparatus described in European patent application no, 88300789 aforementioned.

The ablatable-membrane technique described for the spherically corrective situation in Figs. 2-3 and 4-5, respectively, will be seen to be further applicable to achieve cylindrical correction needed for reducing an astigmatism. For example, if Fig. 2 is taken as depicting vertical-section profiles wherein the cylindrical axis of astigmatism correction is normal to and through axis of 15 of Fig. 2 and wherein Fig. 2 is no longer understood to depict a thickness profile of revolution, but rather a generally V-shaped channel profile extending along a diametral alignment with respect to ring 19, the described exposure course will achieve a cylindrical reduction of cornea curvature; and if ring 19 is preset in rotation about axis 15 such that the cylindrical reduction is oriented to accord with the diagnosed orientation of the patient's astigmatism, then the astigmatism can be reduced to a prescribed diopter-reducing extent merely by correct selection of the maximum thickness of the thickness-characterized ablatable membrane 16. In Fig. 1, an arrow indicator 28 on ring 19 can be brought by ring rotation into register against an azimuth scale 29 on device 10, to enable precise setting of the orientation for an astigmatism-reducing procedure.

In the embodiment thus far described, the thickness-characterized membrane 16 is designed to achieve varying spot-size transmission of stroma-ablating radiation, via a substrate which is transparent to the involved radiation and in this situation, it is particularly convenient to embody the substrate and its ablatable membrane in a circular ring which is self-centering with respect to the optical size of the eye. But requisite transparent substrate materials may prove to be relatively expensive.

The fragmentary diagram of Fig. 1A indicates that substrate expense need not be a problem, in the alternative event of using a plane mirror 30 as the substrate which carries a thickness-characterized ablatable membrane 16'. In Fig. 1A, mirror 30 is part of a ring element 31 which is insertably located in a circular opening in supporting frame structure 32, the latter being a fixed attachment to the laser housing. Mirror 30 is shown inclined at 45° to the incident laser beam so as to fold the same for vertically downward surgical delivery to the cornea 14, to the progressively varying spot-size extent determined by ablation of membrane 16' in the course of a given surgical procedure.

Before incidence with the membrane-coated surface of element 31, the laser beam will be understood to be of homogeneously distributed flux density across its circular section, which may again be of 5 or 6 mm diameter for a myopia-reducing procedure. But at incidence with the membrane-coated surface, the area of incidence is an ellipse wherein the minor axis is horizontal; and the thickness profile at the minor axis will be understood to be as depicted in Fig. 2, for a minor-axis extent of 5 mm, the extent shown in Fig. 2. On the other hand, the major-axis extent will be greater, being shown as 7.08 mm in Fig. 6; and the curves 21 - 22' of varying thicknesses, for the respective maximum thicknesses $T_1$, $T_2$ in the major- axis section plane of Fig. 6, are seen to follow the profiles 21-22 of Fig. 2 on a correspondingly expanded scale. In ay case, the elliptical pattern over which membrane thickness is characterized for myopia-reduction in the reflecting situation of Fig. 1A will be understood to proceed from zero thickness to the centre of the ellipse to maximum thickness $T_1$ (or $T_2$) at the perimeter of the ellipse. Thus, in the course of a given myopia-reducing surgical procedure, the initially reflected beam will be a central spot of circular section, and this post will progressively expand as the characterized membrane is progressively ablated, until the full circular section of the incident laser beam is reflected into surgical incidence with the cornea; at this point, the surgical procedure will have accomplished the prescribed myopia reduction at the anterior surface of the cornea.

Since it is important for operation of a reflecting system (as in Fig. 1A) that the elliptical thickness pattern of ablatable membrane 16' be correctively oriented such that the minor axis is horizontal, a keying slot-and-stud engagement is schematically shown at 35 between a point on ring 31 and a reference point on structure 32.

What has been said as to myopia reduction via ablation of a membrane 16' that has been precharacterized in minor-axis and major-axis section planes according to Figs. 2 and 6, respectively, can also be said for hyperopia reduction when membrane 16' has been precharacterized in minor-axis and major-axis section planes according to Figs. 4 and 7, respectively. It will be recalled, however, from previous discussion that a slightly larger circular-section laser beam is desired for the hyperopia-reducing procedure, and it can be clearly seen from Fig. 7 that the ultimate sculpture of a bevel 27' is just as possible for the reflecting procedure of variable-spot size irradiation (Fig. 1A) as for the transmitting procedure of Fig. 1.

The described invention will be seen to have achieved all stated objects and to provide methods and means for more readily and economically performing a laser-ablated recurvature of the cornea. The surgeon's inventory of membrane-coated rings 19 (or 31) may be precharacterized by thicknesses and thickness profiles which can be labeled in terms well understood by all ophthalmic surgeons, namely in diopters or fractions thereof, and qualified as to plus and minus spherical, with or without the cylindrically correcting feature. The rings 19 may be prepared by applying standardized coatings 16 of substrates 17, in thicknesses graduated for successive increments of predetermined ultimate stroma penetrations to achieve given diopter changes; and the uniform-thickness membrane layer may be "cut" to its precharacterizing thickness profile, by laser ablation pursuant to the teachings of one or more of my previously filed European patent applications. For example, under microprocessor control and using the apparatus of Figs. 8/9 or 15/16, respectively of European patent application no. 87306826 one may prepare for inventory a plurality of rings 19 having membranes 16 with the Fig. 2 spherical or cylindrical correcting thickness profiles; and by using the apparatus of Fig. 30 of said European application one may prepare for further inventory a plurality of such rings 19 having membranes 16 with the Fig 4 thickness profiles.

It should be noted that manufacture of thickness-characterized reflection rings 31 for use in Fig. 1A is as simple and straightforward as was the case for transmission rings 19 for use in Fig. 1. Specifically, using the same apparatus of European patent application no. 87306826 as described for characterizing membrane 16 for myopia reduction or for hyperopia reduction, the only difference is that a uniformly thick ($T_1$ or $T_2$) membrane 16' should be inclined at 45° to the incident homogeneous circular beam when precharacterizing the same. Such inclination will enable automatic development of the described profile, with correct elliptical proportioning and orientation, using the keying reference 35.

However, to manufacture rings 31 which are characterized for astigmatism correction, it is necessary, for use in reflecting system of Fig. 1A, to prepare a series of rings which have been precharacterized for astigmatism correction at each of a series of quantized increments of azimuthal orientation. For the purpose of such manufacture, the astigmatism-reducing apparatus of Figs. 15/16 of European patent application no. 87306826 is selectively adjustable for azimuth orientation, and is well suited to prepare such a series of rings 31.

The expression "zero thickness", as applied herein to describe one of the limits of precharacterized thickness profiling of membrane 16 (or 16') is to be understood as a convenient way of stating (together with the expression "maximum thickness") the range of thickness variation involved in precharacterization to achieve a given recurvature profile of the cornea. Thus, for certain purposes, it may be further convenient or desirable to avoid a truly zero thickness, as for example to provide such added uniformly distributed thickness of membrane 16 (of 16') beneath the described profiling as to permit the surgeon to perform a testing of a given membrane 16 (or 16'), for example, prior to its use for sculpture of a cornea. A test exposure of the homogenized beam to the thus-characterized membrane could illustratively enable the surgeon to determine, as with his stop watch, the time required to expose to the extent of ablative decomposition of the added uniform-thickness increment, such time being visually observed to terminate upon initial laser-beam emergence at the region of least-thickness of the membrane. When the surgeon determines this time, he has in effect calibrated his inserted membrane for its rate of penetrating ablation of the membrane material, thus establishing whether he has selected an element 19 (or 31) with a membrane having the correct diopter-changing value which he has prescribed, or whether he should achieve the prescription by selecting a different ring 19 (or 31) with a membrane designed for its incrementally greater or lesser diopter-changing property. Alternatively, an element 19 (or 31) having a membrane 16 (or 16') that is characterized by known uniform thickness may be a calibrating device forming part of the surgeon's inventory, in that his timing of this laser's ability to ablate through the full membrane thickness of such an element will enable him to immediately determine the current ablating efficacy of his laser beam, thus enabling his appropriate allowance for a drop in ablating efficacy, merely by selecting for a given sculpturing procedure an element 19 (31) having a thickness-characterized membrane 16 (16') that was designed for a laser diopter-changing property, i.e., of lesser maximum thickness.

While the invention has been described herein with particular reference to the field of laser ophthalmological surgery using ultraviolet radiation, this application is merely exemplary of the invention which has wider application. Thus, for example, the ablative radiation is not restricted to laser radiation and particularly not to the ultraviolet region of the electromagnetic spectrum (infrared radiation might for example be used for some applications), and the invention is not limited to the field of ophthalmological surgery though it is considered to be especially useful therefor.

## Claims

1. A method of delivering ablative radiation in a predetermined areal flux distribution onto a surface to be ablated, said method comprising introducing into the path of a beam of ablative radiation a member having a predetermined areal thickness distribution and arranging for the beam of radiation to impinge upon said surface after ablation of said member so that the flux delivery distribution is modulated by the thickness distribution of said member.

2. A device for modulating the areal flux distribution within a beam of ablative radiation, said device comprising material opaque to said radiation and having a predetermined areal thickness distribution such that when the device is placed in said beam ablation of said material produces a differential beam breakthrough distribution such as to modulate the areal flux distribution of the breakthrough beam.

3. A method or apparatus for determining the exposure of a surface to ablative radiation in accordance with which there is introduced into a radiation beam a member opaque to the beam radiation and precharacterized to provide differential breakthrough of the beam in different regions of the beam cross-section, the arrangement being such that different regions of a surface exposed to the beam breaking through said member will receive differential beam flux distributions over a predetermined time period.

4. A method or apparatus for modulating the flux distribution delivered by an ablative radiation beam onto a surface, characterized by the imposition into the beam of a member adapted to be ablated by the beam so as to permit beam breakthrough for impingement of the beam onto said surface with different regions of said member requiring different beam exposure time periods for through ablation.

5. As an article of manufacture, an ablatable membrane for expendable use in providing a predetermined ablative sculpture of the optically used central area of the anterior surface of a cornea, said membrane being opaque to ultraviolet radiation and having a central area of varying thickness which ranges from zero thickness at the centre maximum thickness at a predetermined outer limit of said central area.

6. As an article of manufacture, a plane-parallel plate of substrate material that is transparent to ultraviolet radiation, and an ablatable membrane layer carried by said plate for expendable use in providing a predetermined ablative sculpture of the optically used central area of the anterior surface of the cornea, said membrane being opaque to ultraviolet radiation and having a central circular area of varying thickness which is a function of radius between central inner and radially outer limits of the circular area, the thickness ranging from zero at one of said limits to maximum at the other of said limits, said membrane being of such maximum thickness within said circular area as to require, for a given intensity of ultraviolet radiation, a predetermined total exposure to achieve total ablation of said maximum thickness, said predetermined total exposure being that which is predetermined to achieve a given maximum stroma-penetration depth for a given sculpturing recurvature of the anterior surface of a cornea exposed to ultraviolet radiation via transmission through said plate.

7. As an article of manufacture, a substrate having a flat surface which is a reflector of ultraviolet radiation, and an ablatable membrane layer carried by said surface for expendable use in providing a predetermined ablative sculpture of the optically used central area of the anterior surface of the cornea, said membrane being opaque to ultraviolet radiation and having a central elliptical area of varying thickness which is a function of radius between central inner and radially outer limits of the elliptical area, the thickness ranging from zero at one of said limits to maximum at the other of said limits, said membrane being of such maximum thickness within said elliptical area as to require, for a given intensity of ultraviolet radiation, a predetermined total exposure to achieve total ablation of said maximum thickness, said predetermined total exposure being that which is predetermined to achieve a given maximum stroma-penetration depth for a given sculpturing recurvature of the anterior surface of a cornea exposed to ultraviolet radiation via reflection from said surface.

8. The article of claim 6 or claim 7, wherein the thickness variation is an increasing function of radius between said limits, whereby to achieve a myopia-reducing corneal recurvature, or is a decreasing function of radius between said limits, whereby to achieve a hyperopia-reducing corneal recurvature.

9. The article of claim 6, wherein said substrate and membrane are assembled to a surrounding annular supporting frame, with said circular area concentric to said frame.

10. The article of claim 7, wherein said substrate and membrane are assembled to an annular supporting frame with an angular-keying formation local to at least one region of the periphery of said frame.

11. The article of claim 6 or claim 7, wherein the thickness variation is a decreasing function of radius between said limits, whereby to achieve a hyperopia-reducing corneal recurvature, wherein an outer annulus of radially varying thickness is contiguous to the periphery of said area.

12. The method of making the article of claim 6, which comprises preparing said plate with a uniformly thick membrane layer on one plane surface thereof, and exposing a central area thereof to selective ultraviolet radiation and attendant ablative photodecomposition in a volumetric removal of membrane material and with full depth penetration at one locality within said central area, and essentially zero depth penetration at another locality within said central area, such that a predetermined thickness profile is established between said localities.

13. As an article of manufacture, a plane-parallel plate of substrate material that is transparent to ultraviolet radiation, and an ablatable membrane layer carried by said plate for expendable use in providing a predetermined ablative astigmatism-reducing sculpture of the optically used central area of the anterior surface of the cornea, said membrane being opaque to ultraviolet radiation and having a central area of varying thickness which is an increasing function of offset laterally from a diametrically extending geometric axis through the centre of said central area, the thickness ranging from zero along said diametrically extending axis to maximum at equal and opposite lateral limits of offset from said diametrically extending axis, said membrane being of such maximum thickness within said central area as to require, for a given intensity of ultraviolet radiation a predetermined total exposure to achieve total ablation of said maximum thickness, said predetermined total exposure being that which is predetermined to achieve a given maximum stroma-penetration depth for a given sculpturing astigmatism-reducing recurvature of the anterior surface of a cornea exposed to ultraviolet radiation via transmission through said plate.

14. As an article of manufacture, a substrate having a flat surface which is a reflector of ultraviolet radiation, and an ablatable membrane layer carried by said surface for expendable use in providing a predetermined astigmatism-reducing ablative sculpture of the optically used central area of the anterior surface of the cornea, said membrane being opaque to ultraviolet radiation and having a central elliptical area of varying thickness which is an increasing function of offset laterally from a diametrically extending geometric axis through the intersection of major and minor axes of said elliptical area, the thickness ranging from zero along said diametrically extending axis to maximum at equal and opposite limits of offset from said diametically extending axis, said membrane of such maximum thickness within said elliptical area as to require, for a given intensity of ultraviolet radiation, a predetermined total exposure to achieve total ablation of said maximum thickness, said predetermined total exposure being that which is predetermined to achieve a given maximum stroma-peneration depth for a given sculpturing recurvature of the anterior surface of a cornea exposed to ultraviolet radiation via reflection from said surface.

15. An inventory collection or kit comprising a plurality of the articles of claim 6 or claim 7 or claim 13 or claim 14, wherein the plurality comprises at least one article having an ablative membrane of a first and lesser maximum thickness and a second article having an ablatable membrane of a second and greater maximum thickness.

16. An inventory collection or kit comprising a plurality of the articles of claim 6 or claim 7 or claim 13 or claim 14, wherein the plurality comprises at least one article according to one of said claims and at least another article according to another of said claims.

17. The method of making the article of claim 7 which comprises preparing said reflector with a uniformly thick membrane layer on said flat surface, and at an acute angle of incidence directionally exposing a central area thereof to selective ultraviolet radiation and attendant ablative photodecomposition in a volumetric removal of membrane material and with full depth penetration at one locality within said central area, and essentially zero depth penetration at another locality within said central area, such that a predetermined thickness profile is established between said localities.

18. The method of claim 12 or claim 17, in which the ultraviolet radiation is characterized by a beam having circular symmetry about a central axis of incidence with said membrane layer.

19. An inventory collection or kit comprising a plurality of the articles of claim 6 or claim 7 or claim 13 or claim 14, wherein the plural articles have ablatable membranes of different maximum thickness but of similar varying thickness, and at least another such article in which the membrane layer is of predetermined uniform thickness, whereby upon first exposing the uniform thickness membrane to a source of ablating ultraviolet radiation and upon noting the totality of such exposure needed to decompose to the full extent of said predetermined uniform thickness, one is able to calibrate the current ablating efficacy of said source and thereby make appropriate allowance for such efficacy in selecting for cornea sculpture that one of said plural articles which can most nearly achieve a desired cornea curvature change.

20. The method of changing optical properties of an eye by operating solely upon the anterior surface of the cornea of the eye, which method comprises selecting an ablatable membrane that is opaque to ultraviolet radiation and that has a precharacterized thickness which varies between limits of zero thickness at one localized area and maximum thickness at another localized area, selectively applying ultraviolet radiation to the anterior surface of the cornea by way of radiation passage through said membrane, the maximum thickness of said membrane being sufficient to require such maximum ultraviolet radiation exposure for full decomposition thereof as to assure volumetric removal of corneal tissue and with depth penetration into the stroma and to a predetermined curvature profile.

21. The method of claim 20, wherein the ultraviolet radiation is a coherent beam of generally circular section which at least corresponds to the corneal area to be subjected to optical change.

22. The method of claim 21, wherein the beam cross-section is characterized by substantially uniform flux-density distribution.

23. The method of claim 20, wherein the irradiation is continued to the point of substantially complete ablative photodecomposition of the maximum-thickness area of the ablatable membrane.

24. The method of claim 20, wherein the ablatable membrane is a material selected from the group comprising polyimide, Mylar, and poly-(terephthalate).

25. The method of claim 20, in which the ultraviolet radiation is a pulsed excimer-laser radiation.

26. The method of claim 20, in which the selected ablatable membrane is a coating on a substrate that is transparent to ultraviolet radiation.

27. The method of claim 20, in which the selected ablatable membrane is a costing on a substrate which is a reflector of ultraviolet radiation.

28. The method of claim 20 wherein the maximum precharacterized thickness (Tm) of the membrane is substantially equal to the maximum desired depth of ablative penetration of the stroma, times the expression $A_m/A_s$, where for a given intensity of laser-beam strength, $A_m$ is the rate of depth-penerating ablation of the membrane and $A_s$ is the rate of depth-penetrating ablation of the stroma.

29. The method of changing optical properties of an eye by operating solely upon the anterior surface of the cornea of the eye, which method comprises selecting an ablatable membrane that is opaque to ultraviolet radiation and that has a precharacterized thickness which varies between limits of minimum thickness at one localized area and maximum thickness at another localized area, selectively applying ultraviolet radiation to the anterior surface of the cornea by way of radiation impingement upon said membrane for such radiation exposure as is involved in reducing said minimum thickness to zero, whereupon incident ultraviolet radiation commences to pass through to the cornea via the localized area of zero thickness, the maximum thickness of said membrane being sufficient to require such maximum ultraviolet-radiation exposure for full decomposition thereof as to assure volumetric removal of corneal tissue and with depth peneration into the stroma and to a predetermined curvature profile.

30. The method of claim 29, wherein the maximum pre-characterized thickness ($T_m$) of the membrane, less the minimum thickness ($T_o$) of the membrane, is substantially equal to the maximum desired depth of ablative penetration of the stroma, times the expression $A_m/A_s$, where for a given intensity of laser-beam strength,, $A_m$ is the rate of depth-penetrating ablation of the membrane and $A_s$ is the rate of depth-penerating ablation of the stroma.

31. The method of claim 20 or claim 29, in which said one localized area is an outer circle of maximum radius corresponding to the maximum radius of a central circular area of optically changing curvature of the cornea, and in which said other localized area is at the centre of said outer circle.

32. The method of claim 20 or claim 29, in which said other localized area is an outer circle of maximum radius corresponding to the maximum radius of a central circular area of optically changing curvature of the cornea, and in which said one localized area is at the centre of said outer circle.

33. The method of claim 20 or claim 29, in which said one localized area is a diametral line, which line extends across an outer circle corresponding to a central circular area of optically changing curvature of the cornea, in which said line is oriented in accordance with an astigmatic condition to be corrected, and in which said other localized area comprises like but mirror-image areas within said outer circle, and at maximum equal and opposite offset from said diametral line.

34. The method of claim 29, in which elapsed radiation-exposure time is measured for accomplishing the minimum-thickness reduction to zero, whereby, prior to any substantial laser action upon the cornea, it can be known that the currently delivered laser radiation is within predetermined tolerable limits for achievement of the predetermined curvature profile.

35. The method of changing optical properties of an eye by operating solely upon the anterior surface of the cornea of the eye, which method comprises selecting an ablatable membrane that is opaque to radiation from a particular tissue-ablating laser and that has a precharacterized thickness which varies between limits of zero thickness at one localized area and maximum thickness at another localized area, selectively applying the particular tissue-ablating laser radiation to the anterior surface of the cornea by way of radiation passage through said membrane, the maximum thickness of said membrane being sufficient to require such maximum radiation exposure for full decomposition thereof as to assure volumetric removal of corneal tissue and with depth penetration into the stroma and to a predetermined curvature profile.

36. The method of changing optical properties of an eye by operating solely upon the anterior surface of the cornea of the eye, which method comprises selecting an ablatable membrane that is opaque to radiation from a particular tissue-ablating laser and that has a precharacterized thickness which varies between limits of minimum thickness at one localized area and maximum thickness at another localized area, selectively applying the particular tissue-ablating laser radiation to the anterior surface of the cornea by way of radiation impingement upon said membrane for such radiation exposure as is involved in reducing said minimum thickness to zero, whereupon incident tissue-ablating laser radiation commences to pass through to the cornea via the localized area of zero thickness, the maximum thickness of said membrane being sufficient to require such maximum laser-radiation exposure for full decomposition thereof as to assure volumetric removal of corneal tissue and with depth penetration into the stroma and to a predetermined curvature profile.

37. The method of claim 35 or claim 36, in which the radiation is pulsed-laser radiation.

38. The method of claim 35 or claim 36, in which the selected ablatable membrane is a coating on a substrate that is transparent to the particular tissue-ablating radiation, or is a coating on a substrate which is a reflector of the particular tissue-ablating radiation.

# FIG. I.

LASER

HOMOGENIZED LASER BEAM

16 17 18 19
28
29
10
11
15 14
20 VACUUM
12

# FIG. IA.

31
32 30
35
16'
32

# FIG. 2.

$T_2$
$T_1$
15 22
21
3 2 1 0 2 3 mm
2.5 2.5

# FIG. 4

$T_2$
$T_1$
15
26
25
3 2 1 0 1 2 3 mm
2.5 2.5

# FIG. 3.

24 23
14
15

# FIG. 5.

27' 27
14
15

# FIG. 6.

3.54 0 3.54 mm

# FIG. 7.

$T_1$
$T_2$
3.54 0 3.54 mm

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-8 705 496 (SUMMIT TECHNOLOGY, INC.) * Abstract: figures 1,2,6,7; page 15, line 14 - page 23, line 24 * | 1-6,8,9 ,13,14, 18 | A 61 F 9/00 |
| A | | 15,16, 19 | |
| A | EP-A-0 218 427 (F.A. L'ESPERANCE) * Abstract; figures 5,6,7,16 * | 1-3,7,9 -16,19 | |
| A | EP-A-0 207 648 (F.A. L'ESPERANCE) | | |
| A,D | EP-A-0 280 414 (TAUNTON TECHNOLOGIES INC.) | | |
| | | | **TECHNICAL FIELDS SEARCHED (Int. Cl.4)** |
| | | | A 61 F |

The present search report has been drawn up for all claims

| Place of search | Date of completion of the search | Examiner |
|---|---|---|
| THE HAGUE | 02-06-1989 | STEENBAKKER J. |

EPO FORM 1503 03.82 (P0401)